# DEMANDE DE BREVET EUROPEEN

(11) **EP 3 685 818 A1**
(43) Date de publication de la demande: **29.07.2020**
(21) Numéro de dépôt: 18290114.0
(22) Date de dépôt: 26.09.2018
(51) Int. Cl.: A61K 8/37, A61K 8/42, A61K 8/81, A61K 8/86, A61K 8/92, A61Q 9/02, A61Q 19/00, A61K 8/60

(54) **PRÉPARATION POUR LE RASAGE ET L'APRÈS-RASAGE**

(71) Demandeur: Roard, Dany Lilian, 06250 Mougins (FR)
(72) Inventeur: Roard, Dany Lilian, 06250 Mougins (FR)

(57) **Abrégé**

La présente invention est une préparation pour le rasage, hydrosoluble, anhydre (moins de 2% d'eau) donnant des solutions transparentes, non moussantes à fort caractère lubrifiant et conditionnant. Cette préparation est faite pour être utilisée avec un dispositif de rasage électrique (rasoir, tondeuse) à tête lavable (partant, fonctionnant sur batterie d'accumulateurs électrique pour des raisons de sécurité et de praticité), en apportant, pendant le rasage la lubrification adéquate de l'épiderme pour éviter les irritations de l'épiderme, créées par l'appui et le va et vient de la tête de coupe. Le rasage terminé et après un rinçage à l'eau de l'épiderme, cette préparation est capable encore d'assurer le conditionnement de l'épiderme pour un effet après-rasage, sans avoir à remettre de produit (action adoucissante, hydratante, rafraîchissante, tonifiante, astringente, anti-inflammatoire et odoriférante). Le caractère mouillant donné à la solution dans l'eau de cette préparation, permet un nettoyage instantané de la tête de rasage du dispositif. Pour satisfaire à de bonnes conditions de rasage avec un dispositif de rasage électrique, cette préparation est répartie (200 à 600 mg) sur le visage ou sur les parties à raser après avoir mouillé abondamment le visage ou les parties à raser. Le rasage terminé et sans avoir à en remettre, l'épiderme reste adouci, hydraté, rafraîchi. Compte tenu des faibles quantités nécessaires pour un usage, les emballages peuvent être de petit volume, ce qui est bien sûr intéressant en voyage, en vacances. Destiné aux utilisateurs de dispositifs de rasage électrique, cette préparation a aussi la capacité de pouvoir être utilisée avec un dispositif de rasage manuel (rasoir mécanique).

## Description

La présente invention est une préparation pour le rasage utilisable, soit avec un dispositif de rasage électrique (rasoir, tondeuse) à tête lavable (et partant, fonctionnant sur batterie d'accumulateurs électriques pour des raisons de sécurité et de praticité), soit avec un rasoir mécanique (dit rasoir à main) en apportant, pendant le rasage la lubrification adéquate de l'épiderme pour éviter irritations et coupures, et, le rasage terminé et après un rinçage à l'eau de l'épiderme, capable encore d'assurer le conditionnement de l'épiderme, pour un effet Après rasage sans avoir à remettre de produit : (action adoucissante, hydratante, rafraîchissante, tonifiante, astringente, anti-inflammatoire et odoriférante) .

Cette préparation pour le rasage, capable de permettre tant le « rasage électrique » que le « rasage mécanique » et assurant encore la fonction d'après-rasage, sans avoir à en rajouter, est une préparation hydrosoluble, anhydre (moins de 2 % d'eau), non-moussante.

Cette préparation de rasage, que nous appelons encore Fluide de rasage, est faite pour un usage mixte électrique ou bien mécanique, utilisant d'infimes quantités (pour un visage : 200 à 600 mg, selon les types de barbes, l'habitude de l'utilisateur et le type de rasoir) apportées sur les parties à raser, préalablement abondamment mouillées, permet l'emploi, pour un nombre d'utilisations équivalent, d'emballages de capacité 5 à 8 fois plus petit que ceux habituellement requis, ce qui entraîne une diminution des coûts de production et ce qui réduit très considérablement l'empreinte carbone.

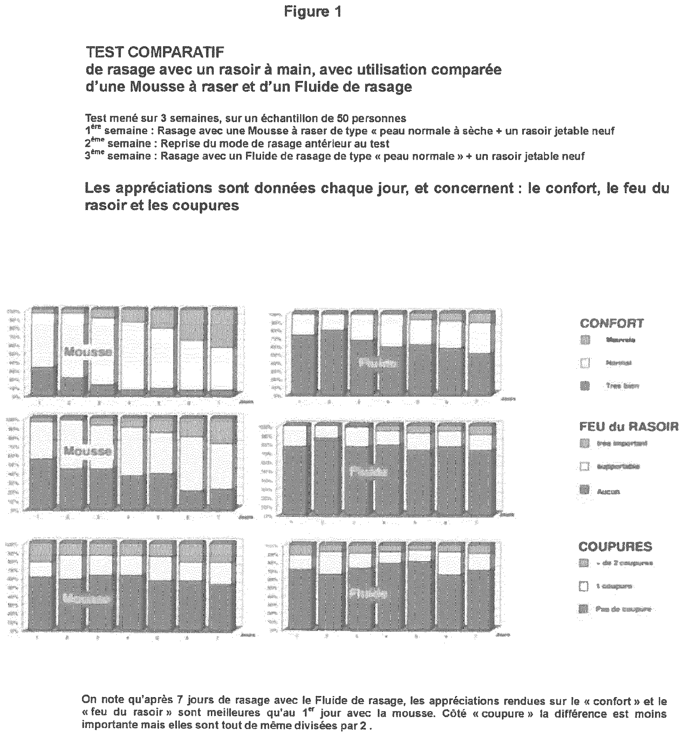

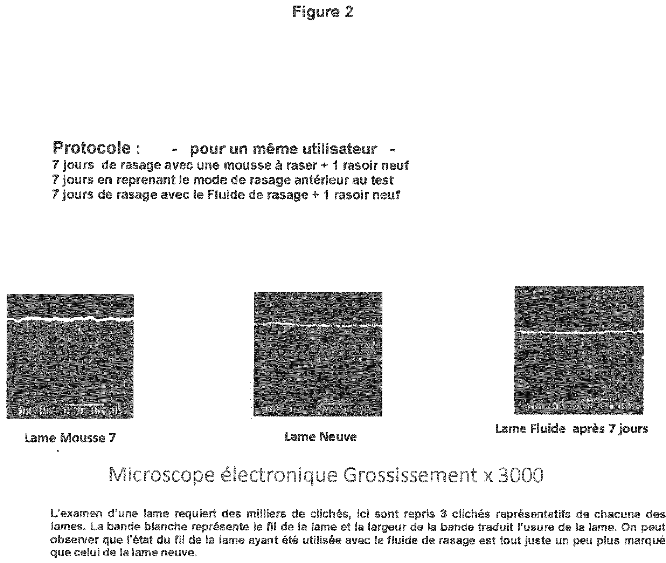

D'autre part, les tests et mesures effectués avec un dispositif de rasage mécanique, ***(******figures 1*** ***et*** ***2******, voir pages 11 et 12)*** font bien ressortir l'intérêt d'une bonne lubrification pour assurer la protection de l'épiderme lors de l'opération de rasage : les dermatologues y voient un autre moyen de se protéger des pathologies liées au rasage, les cardiologues eux s'intéressent à la quasi absence de coupures lors du rasage quotidien pour leurs patients souvent sous anticoagulants, les chirurgiens au caractère non-moussant, protecteur et assainissant qui permet de préparer aisément la zone préalablement à la chirurgie à effectuer.
Ceci explique qu'ouvrir cette protection aux utilisateurs des dispositifs de rasage électrique est tout à fait pertinent. En effet, dès que l'utilisateur appuie la tête du rasoir, la grille de protection placée sur les têtes de coupe des dispositifs de rasage électrique, (entre autres, celles des «rasoirs électriques»), celles-ci se comportent comme une rape sur l'épiderme, du fait de l'altemance de points durs (la grille) et de trous (chargés de laisser passer les poils). De fait, l'irritation est bien réelle et souvent visible et pénible sur le cou, le menton.
Pour autant, très peu d'utilisateurs de «rasoirs électriques» utilisent un produit pour limiter, voire, empêcher ces irritations. Des huiles sont proposées, le plus souvent aux fins de lubrifier le frottement des couteaux sur les grilles, diminuant ainsi leur température et conservant ainsi, plus longtemps, le bon affutage des couteaux rotatifs ou vibrants, tandis que la quantité d'huile arrivant sur l'épiderme et limitant les frottements avec la tête de rasage, procure à l'utilisateur plus de confort.

Le brevet WO9847474 A1 de la Sté Philips, décrit une émulsion aqueuse qui permet d'obtenir d'excellentes conditions de rasage avec un lubrifiant, le 2,2,4,4,6,8,8 - heptamethylnonate, mis en émulsion avec le n décyl glucoside.
Cette émulsion remplit un réservoir souple placé à l'intérieur du rasoir.
Ce dispositif produit-réservoir, se distingue tout à fait de notre préparation, en ce que,
**premièrement**, s'agissant d'une émulsion aqueuse, le volume occupé par ce lubrifiant se trouve de fait 3 à 5 fois plus important et, par là même, qu'à volume identique utilisé pour un rasage, il permet beaucoup moins de rasages que la présente préparation ;
**deuxièmement**, qu'il demande une adaptation du rasoir pour recevoir le réservoir souple mentionné, alors qu'avec la préparation objet de la présente invention, la quasi-totalité des rasoirs d'aujourd'hui, étant à tête lavable et fonctionnant de ce fait sur batterie, peuvent être utilisés sans risque, ni difficulté ;
**troisièmement**, l'émulsion mentionnée ne fait pas état de propriétés de conditionnement de l'épiderme pour un effet après-rasage ;
**quatrièmement**, il n'est pas non plus revendiqué de capacité pour cette émulsion de pouvoir être utilisée avec un rasoir mécanique ;
**cinquièmement**, la préparation objet de la présente demande d'invention, est un fluide parfaitement hydrosoluble, **anhydre** (moins de 2 % en eau) qui présente une grande stabilité dans le temps. Ce qui est plus difficilement le cas d'une émulsion qui requiert, en plus et pour cela, l'emploi de conservateurs, alors que la présente préparation n'en nécessite aucun.

Les brevets : français 9610088 et européen EP 0823253B1 que j'ai précédemment déposés soulignent bien l'intérêt apporté par une lubrification hydrosoluble lors du rasage effectué avec un rasoir mécanique : infime quantité pour conduire un rasage, petit volume de produit permettant d'exécuter un grand nombre de rasage, absence d'aérosol et partant des contributions à la pollution qui en découlent. **Toutefois, il y est bien précisé que le brevet ne s'intéresse qu'au seul rasage mécanique**, pour la raison qu'alors en 1996 et 97, c'était tout simplement une hérésie d'envisager de se raser le visage mouillé d'une solution lubrifiante aqueuse en utilisant un système électrique. Certain accident domestique survenu deux décennies auparavant, étant dans toutes les mémoires. Dès lors, le rasage électrique s'effectuait sans aucun produit, et cela en était même un crédo.
Aujourd'hui, vingt ans plus tard, la quasi-totalité des rasoirs sont proposés sur batterie, donc basse tension et qui plus est « à tête lavable » ce qui impose que le dispositif fonctionne hors tout branchement sur le secteur pour des raisons de sécurité. Et si l'habitude de ne pas utiliser de produit facilitant le rasage électrique reste lourdement ancrée, ce n'est plus un risque de se raser avec une solution lubrifiante aqueuse, il n'est plus nécessaire d'utiliser un rasoir dédié, comme décrit dans le brevet Wo9847474 A1, encore fallait-il y penser.

Nous avons donc travaillé sur une évolution de la formule de notre préparation qui lui permettrait de satisfaire aux conditions d'un rasage effectué avec un rasoir électrique et d'en circonscrire les risques.
En prenant en compte, pour ce faire, que, s'agissant de la tête de rasage d'un rasoir électrique, sa surface de contact est augmentée 3 à 6 fois par rapport à celle des rasoirs mécaniques jetables ou système, tandis que le poids du dispositif déplacé sur le visage est 10 à 20 fois supérieur à celui d'un rasoir à main, la cinétique de l'appareil, sur le visage ou les parties à raser, variant selon l'utilisateur et ses gestes. Cela a pour conséquences, d'engendrer des pressions exercées sur l'épiderme 3 à 5 fois supérieures à celles exercées par un rasoir mécanique, d'une part,
d'autre part, s'ajoute le problème de l'élimination du produit de la coupe des poils qui se retrouvent à l'intérieur des têtes de rasage, qu'il est nécessaire de pouvoir traiter facilement, d'un simple rinçage à l'eau et dans des conditions d'hygiène satisfaisantes.
La préparation ayant déjà par la présence de ses huiles essentielles une capacité assainissante, restait à traiter du pouvoir mouillant de la préparation mise en solution dans l'eau apportée sur l'épiderme. Celui-ci s'avérant insuffisant pour pratiquer un enlèvement aisé de la bouillie de poils garnissant l'intérieur de la tête, devait être retravaillé pour qu'un simple rinçage à l'eau des têtes de rasage, permette un nettoyage satisfaisant.

Ces constatations faites il a été jugé nécessaire pour satisfaire aux conditions particulières d'un rasage effectué avec un rasoir électrique :
A - d'accroitre la résistance à la pression du film lubrifiant mis en place sur l'épiderme. Au moyen d'une augmentation de leur pourcentage dans la formule, de leur substantivité avec l'épiderme et aussi par l'incorporation de nouveaux composés lubrifiants. Ce qui a été fait.
B - d'augmenter le pouvoir mouillant de la préparation mise en solution dans l'eau. Ce qui a été obtenu par l'augmentation du % en tensio-actifs.
Ainsi, avec la solution aqueuse mouillante et lubrifiante imprégnant la bouillie de poils coupés qui se retrouve dans la tête de rasage du rasoir, le nettoyage s'effectue facilement par simple rinçage à l'eau.

Lors d'un rasage exécuté avec un dispositif de coupe électrique, l'intérêt d'une lubrification en milieu aqueux est bien réel. Ainsi, la présente préparation, apportée sur l'épiderme préalablement abondamment mouillé, permet-elle, avec seulement 200 à 600 mg pour le rasage d'un visage, d'assurer d'excellentes conditions de confort et d'absence d'irritations. Les tests effectués montrent que la résistance à l'avancement de la tête du rasoir est réduite de 20 % avec un film huileux (500 mg répartis sur 300 cm2 d'épiderme), alors qu'elle est réduite de près de 50 %, lorsque sur cette même surface, préalablement mouillée, sont répartis, cette fois, 500 mg de la présente préparation. En conséquence, l'utilisation de la présente préparation lubrifiante, lors du rasage avec un rasoir électrique, est tout à fait intéressante car elle diminue très considérablement l'abrasion générée par les frottements de la tête de rasage du rasoir électrique sur l'épiderme, empêchant ainsi la survenue d'irritations. Les conditions de sécurité étant par ailleurs remplies si le rasoir électrique est à tête lavable. Ce qui devra être visiblement indiqué sur les emballages.

Cette préparation satisfaisant à une utilisation avec, soit un dispositif de rasage électrique, soit mécanique (l'un ou l'autre) et dont la formulation assure, outre la fonction de produit de rasage, celle de produit d'après rasage en laissant sur l'épiderme, le rasage terminé, un réel effet après rasage, se caractérise en ce que :
pour servir une fonction très protectrice de produit de rasage, elle contient, d'une part, des agents lubrifiants, émollients, astringents, mouillants, aux fins de ne plus avoir d'irritations liées aux effets abrasifs des têtes de rasage et de permettre, le rasage terminé, le nettoyage quasi-instantané des têtes de rasage par simple rinçage à l'eau,
et d'autre part,
en ce que, pour servir la fonction de produit après-rasage, elle contient des agents anti-déshydratation, adoucissants, assainissants, rafraîchissants et odoriférants, par-delà le temps du rasage et un éventuel rinçage à l'eau.

Plus particulièrement, la préparation, objet de la présente demande, est caractérisée par sa formule, anhydre, (c'est-à-dire contenant moins de 2% de son poids en eau), hydrosoluble, non-moussante,

Ainsi, Cette préparation hydrosoluble est caractérisée par le fait qu'elle est conçue, pour être utiliséeavec un dispositif de rasage à mouvement électrique pour, une fois massée en très petite quantité (200 à 600 mg) sur le visage (lequel aura été préalablement abondamment mouillé), former une émulsion aqueuse, non-moussante et transparente aux capacités lubrifiante et conditionnante pour l'épiderme, aux fins :
a) de protéger l'épiderme des utilisateurs de l'abrasion causée par les passages répétés et la pression exercée par les têtes de ces dispositifs de rasage électrique,
b) pour, une fois le rasage terminé et sans qu'il soit besoin d'en remettre, assurer un réel effet après-rasage, adoucissant, hydratant et odoriférant sur l'épiderme.
c) pour permettre et faciliter le nettoyage parfait des têtes de rasage, assorti d'un effet assainissant.

Ce, parce que la quasi-totalité de ces utilisateurs de dispositifs de rasage électrique opèrent aujourd'hui sans aucun produit de protection, alors que l'abrasion est bien réelle et que cette nouvelle protection, outre l'absence d'irritations qu'elle procure, leur permet d'appuyer davantage les têtes de rasage, sans dommage pour l'épiderme mais avec une finition de rasage supérieure et un réel effet après rasage.

Cette préparation hydrosoluble est caractérisée par le fait que, lors de son utilisation avec un dispositif de rasage à mouvement électrique, elle sert à la fois et sans avoir à en remettre, les besoins de protection et de conditionnement de l'épiderme qu'aurait apporté l'utilisation successive d'un produit de rasage très lubrifiant, capable d'assurer une parfaite protection de l'épiderme pendant la durée de l'opération de rasage et le rasage terminé, le bénéfice d'hydratation, de douceur propre à l'application sur l'épiderme d'un après-rasage.
Pour cela cette préparation est caractérisée par le fait que sa formule est à double effet, qu'elle sert à la fois de produit de rasage et d'après-rasage.

**Que pour satisfaire aux fonctions de produit de rasage,**
des agents émollients pour une concentration totale en poids dans la formule comprise entre 1 et 30 %, pris parmi les produits suivants, lesquels peuvent respectivement apparaître dans la formule de la présente préparation dans les fourchettes données en % poids ci-après précisées, tout autant que la somme de leur participation en poids dans la formule reste bien dans la fourchette donnée aux agents émollients :

| | |
|---|---|
| • Alcools lauriques polyéthoxylés : | 0.5 à 25 % |
| • Copolymères d'éthylène-propylène | 0.5 à 25 % |
| • Polyéthylènes-glycols | 1 à 30 % |
| • Huile d'abricot | 0.5 à 25 % |
| • Huile d'amande douce | 0.5 à 25 % |
| • Huile de ricin | 0.5 à 25 % |
| • Huile de bourrache | 0.5 à 25 % |

des agents lubrifiants pour une concentration totale en poids dans la formule comprise entre 1 et 90 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents lubrifiants :

| | |
|---|---|
| • PEG n - esters d'acides gras | 3 à 50 % |
| • Alkyls stéarates | 1 à 15 % |
| • PEG n | 1 à 30 % |
| • Méthyl siloxan | 1 à 25 % |
| • Oxydes de polyéthylène | 0.1à 4 % |
| • Gomme xanthane | 0.05 à 2 % |
| • Polyquaternium-7 | 0.05 à 2 % |
| • Huiles de paraffine | 1 à 50 % |

des agents odoriférants pour une concentration totale en poids dans la formule comprise entre 1 et 30 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents odoriférants :

| | |
|---|---|
| • HE d'orange | 0.1 à 20 % |
| • HE de Coriandre | 0.1 à 20 % |
| • HE de Lavande | 0.1 à 20 % |
| • HE de Menthe | 0.1 à 20 % |
| • HE de Vétiver | 0.1 à 20 % |
| • HE de Romarin | 0.1 à 20 % |
| • HE de Citron | 0.1 à 20 % |
| • HE de Limette | 0.1 à 20 % |
| • HE d'Eucalyptus | 0.1 à 20 % |
| • HE de Mandarine | 0.1 0 20% |

des agents astringents pour une concentration totale en poids dans la formule comprise entre 0.5 et 20 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents astringents :

| | |
|---|---|
| • Extrait de Romarin | 0.5 à 20 % |
| • Extrait de Bouleau | 0.5 à 20 % |
| • Extrait de Lavande | 0.5 à 20 % |

des agents mouillants pour une concentration totale en poids dans la formule comprise entre 1 et 50 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents mouillants :

| | |
|---|---|
| • Copolymères d'éthylène-propylène | 0.5 à 25 % |
| • Alcools lauriques polyéthoxylés | 0.5 à 25% |
| • Polyéthylènes glycol-Esters d'Acides gras | 3 à 50 % |
| • Alkyls-glucosides | 0.5 à 20 % |

et,
d'autre part, la préparation est caractérisée par le fait **que pour satisfaire à la fonction de produit d'après rasage** - avec la seule quantité déposée sur l'épiderme avant le rasage, celui-ci étant effectué soit avec un rasoir électrique, soit avec un rasoir à mains - et après que l'épiderme ait été remouillé éventuellement plusieurs fois -
**cette préparation contient pour assurer ces effets** substantifs :
des agents adoucissants, surgraissants pour une concentration totale en poids dans la formule comprise entre 0.5 et 30 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents adoucissants, surgraissants et anti-déshydratation :

| | |
|---|---|
| • Alcanolamides de coprah | 0.5 à 30 % |
| • Huile d'Argan | 0.5 à 30 % |
| • Huile de tournesol | 0.5 à 30 % |
| • Allantoin | 0.2 à 5 % |
| • Alkyls stéarates | 1.0 à 15 % |

des agents rafraîchissants pour une concentration totale en poids dans la formule comprise entre 0.5 et 5 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents rafraîchissants :

| | |
|---|---|
| • Menthol | 0.2 à 5 % |
| • Lactate de menthol | 0.2 à 5 % |
| • HE de menthe | 0.1 à 5 % |
| • HE d'eucalyptus | 0.1 à 5 % |

des agents tonifiants pour une concentration totale en poids dans la formule comprise entre 0.5 et 5 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents tonifiants :

| | |
|---|---|
| • HE de Thym | 0.2 à 5 % |
| • HE de romarin | 0.1 à 5 % |
| • Escin | 0.1 à 5 % |

des agents anti-inflammatoires pour une concentration totale en poids dans la formule comprise entre 0.5 et 5 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents anti-inflammatoires :

| | |
|---|---|
| • Allantoin | 0.2 à 5 % |
| • Menthol | 0.2 à 5 % |
| • HE de menthe | 0.1 à 5 % |
| • Huile de Calendula | 1.à 50 % |

des agents assainissants pour une concentration totale en poids dans la formule comprise entre 0.5 et 20 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents assainissants :

| | |
|---|---|
| • Ceux d'un mélange d'huiles essentielles | 1 à 5 % |

des agents anti-déshydratation pour une concentration totale en poids dans la formule comprise entre 1.0 et 30 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents anti-déshydratation :

| | |
|---|---|
| • Isononanoate de cétéaryle | 1 à30 % |
| • Huile de Barbasse | 1. à 30 % |
| • Huile d'Argan | 1 à 30 % |
| • Huile d'amande douce | 0.5 à 25 % |

des agents odoriférants pour une concentration totale en poids dans la formule comprise entre 1.0 et 30 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents odoriférants :

| | |
|---|---|
| • HE d'orange | 0.1 à 20 % |
| • HE de Coriandre | 0.1 à 20 % |
| • HE de Lavande | 0.1 à 20 % |
| • HE de Menthe | 0.1 à 20 % |
| • HE de Vétiver | 0.1 à 20 % |
| • HE de Romarin | 0.1 à 20 % |
| • HE de Citron | 0.1 à 20 % |
| • HE de Limette | 0.1 à 20 % |
| • HE d'Eucalyptus | 0.1 à 20 % |
| • HE de Mandarine | 0.1 à 20 % |

des agents antioxydants pour une concentration totale en poids dans la formule comprise entre 0.5 et 5 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents antioxydants :

| | |
|---|---|
| • Tocophérol | 0.5 à 5 % |
| • Huile d'argan | 1à 5 % |
| • Huile de Barbasse | 1à 5 % |

Etant entendu que la somme des pourcentages des différents agents : émollients, lubrifiants, odoriférant, astringents..... et antioxydants de la présente préparation, n'excède pas 100 %.

Caractéristique de la présente préparation hydrosoluble : elle peut aussi être utilisée avec un dispositif de rasage mécanique, dit « rasoir à main », et que, lors de cette utilisation, elle sert à la fois et sans avoir à en remettre, les besoins de protection et de conditionnement de l'épiderme qu'aurait apporté l'utilisation successive d'un produit de rasage très lubrifiant, capable d'assurant une parfaite protection de l'épiderme pendant la durée de l'opération de rasage et le rasage terminé, elle apporte le bénéfice d'hydratation, de douceur propre à l'application sur l'épiderme d'un après-rasage.

Caractéristique de la présente préparation : l'effet substantif avec l'épiderme des composés lubrifiants qu'elle contient, fait qu'ils demeurent sur l'épiderme pendant toute la durée du rasage et que si la lubrification vient à diminuer, du fait de la perte d'eau liée aux conditions de rasage, il suffit alors de remouiller le visage en exerçant un petit massage de l'épiderme de quelques seconde pour recréer une bonne lubrification et ainsi mener le rasage en gardant sur toute sa durée une bonne protection lubrifiante.

Caractéristique de la présente préparation, le fait que sa solution aqueuse mouillante et lubrifiante, mise en place sur le visage ou sur les parties à raser, imprègne aussi la bouillie de poils coupés qui se retrouve dans la tête de rasage du rasoir, dès lors, le nettoyage des têtes de rasage s'effectue facilement par simple rinçage à l'eau.

Caractéristique de la présente préparation, objet de la présente demande de brevet, une fois mise en solution sur un visage mouillé (200 à 600 mg selon la barbe et les gestes de l'utilisateur), sa capacité à satisfaire pleinement aux bonnes conditions d'un rasage effectué soit à l'aide d'un dispositif de rasage électrique, soit l'aide d'un dispositif de rasage mécanique et le rasage terminé d'assurer à l'épiderme, sans avoir à en rajouter, un réel effet après rasage.

Les exemples suivants représentatifs de la composition de cette préparation servant à réaliser le rasage et l'après-rasage, pour l'un ou l'autre des modes de rasage, électrique ou mécanique, sont donnés, ci-après, pour illustrer la procédure de fabrication. Ces exemples ne doivent pas être considérés comme limitatifs.

### Exemple 1

| Produit de rasage & d'après rasage, pour le rasage électrique & mécanique | |
|---|---|
| Désignation CTFA - **Formule 0122097** | % Poids |
| Huile d'abricot | 2.00 |
| Polyéthylène Glycol n - Esters d'acide gras | 35.00 |
| Polyquaternium 7 | 0.10 |
| Mélange d'huiles essentielles * | 16.39 |
| Huile d'Argan | 2.00 |
| PEG n | 10.00 |
| Alcool Laurique (0Et)n | 3.40 |
| Copolymère d'éthylène-Propylène | 8.00 |
| Alcanolamide de coprah | 8.00 |
| Isononanoate de cetearyle | 13.00 |
| Colorant E131 | 0.01 |
| Alkyl Stéarate | 2.00 |
| Escin | 0.10 |
| | 100.00 |
| (*) Composition du Mélange d'huiles essentielles : HE Menthe 17.32 % ; HE Lavande 18.73 % ; HE Eucalyptus 20.62 % ; Composition parfumée 43.33 %. | |

*Dans le mélange d'huiles essentielles, ajouter sous agitation et successivement : le PEGn, le PEGm-Esters d'acides gras, l'Alcool Laurique polyéthoxylé, le Copolymère d'éthylène-propylène, les huiles végétales, l'Isononanoate de cétéaryle, Verser alors l'Alcanolamide de Coprah, l'Escin et le Polyquaternium, enfin la solution de colorant sous agitation vive.*

### Exemple 2

| Produit de rasage & d'après rasage, pour le rasage électrique & mécanique | |
|---|---|
| Désignation CTFA - **Formule 0222097** | % Poids |
| Huile de Ricin | 11.00 |
| Méthyl siloxan | 2.00 |
| Huile d'Amande douce | 2.00 |
| Huile de tournesol | 8.00 |
| Huile de barbasse | 2.00 |
| PEG n | 10.00 |
| Alcool Laurique (0Et)n | 15.00 |
| Mélange d'huiles essentielles * | 5.00 |
| Polyéthylène Glycol n - Esters d'acide gras | 20.00 |
| Copolymère d'éthylène-Propylène | 8.00 |
| Alcanolamide de coprah | 4.00 |
| Isononanoate de cetearyle | 9.00 |
| Colorant E131 | 0.01 |
| Alkyl Stéarate | 1.99 |
| Allantoin | 1.00 |
| Menthe cristallisée | 1.00 |
| Composition du Mélange d'huiles essentielles : HE Vetiver 5.00 % ; HE Romarin 15.00 % ; HE Thym 20.00 % ; HE de Mandarine 20 % ; Composition parfumée 40.00 %. | 100.00 |

*Dans le mélange d'huiles essentielles, ajouter sous agitation le menthol cristallisé puis, successivement: Le PEG n, le PEG n* - *Esters d'acides gras, l'Alcool Laurique polyéthoxylé, le Copolymère d'éthylène-propylène, les huiles végétales, l'Isononanoate de cétéaryle. Verser alors l'Alcanolamide de Coprah, Ajouter l'Allantoin, enfin la solution de colorant sous agitation vive.*

## Revendications

1. Cette préparation hydrosoluble est **caractérisée par le fait qu'**elle est conçue, pour être utilisée avec un dispositif de rasage à mouvement électrique pour, une fois massée en très petite quantité (200 à 600 mg) sur le visage (lequel aura été préalablement abondamment mouillé), former une émulsion aqueuse, non-moussante et transparente aux capacités lubrifiantes et conditionnante pour l'épiderme, aux fins :
- de protéger l'épiderme des utilisateurs de l'abrasion causée par les passages répétés et la pression exercée par des têtes de ces dispositifs de rasage électriques,
- pour, une fois le rasage terminé et sans qu'il soit besoin d'en remettre, assurer un réel effet après rasage, adoucissant, hydratant et odoriférant sur l'épiderme.
- pour permettre et faciliter le nettoyage parfait des têtes de rasage, assorti d'un effet assainissant.

2. pour cela cette préparation, selon la revendication 1, est **caractérisée, par le fait que** sa formule est à double effet, c'est-à-dire qu'elle sert à la fois de produit de rasage et d'après rasage, que, **pour satisfaire aux fonctions de produit de rasage, elle contient** :
des agents émollients pour une concentration totale en poids dans la formule comprise entre 1 et 30 %, pris parmi les produits suivants, lesquels peuvent respectivement apparaître dans la formule de la présente préparation dans les fourchettes données en % poids ci-après précisées, tout autant que la somme de leur participation en poids dans la formule reste bien dans la fourchette donnée aux agents émollients :
| | |
|---|---|
| • Alcools lauriques polyéthoxylés : | 0.5 à 25 % |
| • Copolymères d'éthylène-propylène | 0.5 à 25 % |
| • Polyéthylènes-glycols | 1 à 30 % |
| • Huile d'abricot | 0.5 à 25 % |
| • Huile d'amande douce | 0.5 à 25 % |
| • Huile de ricin | 0.5 à 25 % |
| • Huile de bourrache | 0.5 à 25 % |
des agents lubrifiants pour une concentration totale en poids dans la formule comprise entre 1 et 90 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents lubrifiants :
| | |
|---|---|
| • PEG n - esters d'acides gras | 3 à 50 % |
| • Alkyls stéarates | 1 à 15 % |
| • PEG n | 1 à 30 % |
| • Méthyl siloxan | 1 à 25 % |
| • Oxydes de polyéthylène | 0.1 à 4 % |
| • Gomme xanthane | 0.05 à 2 % |
| • Polyquaternium-7 | 0.05 à 2 % |
| • Huiles de paraffine | 1 à 50 % |
des agents odoriférants pour une concentration totale en poids dans la formule comprise entre 1 et 30 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents odoriférants :
| | |
|---|---|
| • HE d'orange | 0.1 à 20 % |
| • HE de Coriandre | 0.1 à 20 % |
| • HE de Lavande | 0.1 à 20 % |
| • HE de Menthe | 0.1 à 20 % |
| • HE de Vétiver | 0.1 à 20 % |
| • HE de Romarin | 0.1 à 20 % |
| • HE de Citron | 0.1 à 20 % |
| • HE de Limette | 0.1 à 20 % |
| • HE d'Eucalyptus | 0.1 à 20 % |
| • HE de Mandarine | 0.1 0 20 % |
des agents astringents pour une concentration totale en poids dans la formule comprise entre 0.5 et 20 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents astringents :
| | |
|---|---|
| • Extrait de Romarin | 0.5 à 20 % |
| • Extrait de Bouleau | 0.5 à 20 % |
| • Extrait de Lavande | 0.5 à 20 % |
des agents mouillants pour une concentration totale en poids dans la formule comprise entre 1 et 50 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents mouillants :
| | |
|---|---|
| • Copolymères d'éthylène-propylène | 0.5 à 25 % |
| • Alcools lauriques polyéthoxylés | 0.5 à 25% |
| • Polyéthylènes glycol-Esters d'Acides gras | 3 à 50 % |
| • Alkyls-glucosides | 0.5 à 20 % |
et,
d'autre part, la préparation est **caractérisée par le fait que pour satisfaire à la fonction de produit d'après rasage** - avec la seule quantité déposée sur l'épiderme avant le rasage, celui-ci étant effectué soit avec un rasoir électrique, soit avec un rasoir à main et après que l'épiderme ait été remouillé éventuellement plusieurs fois - **cette préparation contient pour assurer ces effets substantifs :**
des agents adoucissants, surgraissants pour une concentration totale en poids dans la formule comprise entre 0.5 et 30 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents adoucissants, surgraissants et anti-déshydratation :
| | |
|---|---|
| • Alcanolamides de coprah | 0.5 à 30 % |
| • Huile d'Argan | 0.5 à 30 % |
| • Huile de tournesol | 0.5 à 30% |
| • Allantoin | 0.2 à 5 % |
| • Alkyls stéarates | 1.0 à 15 % |
des agents rafraîchissants pour une concentration totale en poids dans la formule comprise entre 0.5 et 5 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents rafraîchissants :
| | |
|---|---|
| • Menthol | 0.2 à 5 % |
| • Lactate de menthol | 0.2 à 5 % |
| • HE de menthe | 0.1 à 5 % |
| • HE d'eucalyptus | 0.1 à 5 % |
des agents tonifiants pour une concentration totale en poids dans la formule comprise entre 0.5 et 5 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents tonifiants :
| | |
|---|---|
| • HE de Thym | 0.2 à 5 % |
| • HE de romarin | 0.1 à 5 % |
| • Escin | 0.1 à 5 % |
des agents anti-inflammatoire pour une concentration totale en poids dans la formule comprise entre 0.5 et 5 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents anti-inflammatoire :
| | |
|---|---|
| • Allantoin | 0.2 à 5 % |
| • Menthol | 0.2 à 5 % |
| • HE de menthe | 0.1 à 5 % |
| • Huile de Calendula | 1.à 50 % |
des agents assainissants pour une concentration totale en poids dans la formule comprise entre 0.5 et 20 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents assainissants :
| | |
|---|---|
| • Ceux d'un mélange d'huiles essentielles | 1 à 5 % |
des agents anti-déshydratation pour une concentration totale en poids dans la formule comprise entre 1.0 et 30 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents anti-déshydratation :
| | |
|---|---|
| • Isononanoate de cétéaryle | 1 à30 % |
| • Huile de Barbasse | 1. à 30 % |
| • Huile d'Argan | 1 à 30 % |
| • Huile d'amande douce | 0.5 à 25 % |
des agents odoriférants pour une concentration totale en poids dans la formule comprise entre 1.0 et 30 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents odoriférants :
| | |
|---|---|
| • HE d'orange | 0.1 à 20 % |
| • HE de Coriandre | 0.1 à 20 % |
| • HE de Lavande | 0.1 à 20 % |
| • HE de Menthe | 0.1 à 20 % |
| • HE de Vétiver | 0.1 à 20 % |
| • HE de Romarin | 0.1 à 20 % |
| • HE de Citron | 0.1 à 20 % |
| • HE de Limette | 0.1 à 20 % |
| • HE d'Eucalyptus | 0.1 à 20 % |
| • HE de Mandarine | 0.1 à 20 % |
des agents antioxydants pour une concentration totale en poids dans la formule comprise entre 0.5 et 5 %, pris parmi les produits suivants, lesquels peuvent respectivement apparaître dans la formule de la présente préparation dans les fourchettes données en % poids ci-après précisées, tout autant que la somme de leurs participations respectives en % poids reste bien dans la fourchette donnée ci-dessus aux agents antioxydants :
| | |
|---|---|
| • Tocophérol | 0.5 à 5 % |
| • Huile d'argan | 1 à 5 % |
| • Huile de Barbasse | 1 à 5 % |
Etant entendu que la somme des pourcentages des différents agents : émollients, lubrifiants, odoriférants, astringents.....et antioxydants de la présente préparation, n'excède pas 100 %.

3. Cette préparation hydrosoluble, selon la revendication 2, est **caractérisée par le fait qu'**elle peut aussi être utilisée avec un dispositif de rasage mécanique, dit « rasoir à main », et que, lors de cette utilisation, elle sert à la fois et sans avoir à en remettre, les besoins de protection et de conditionnement de l'épiderme qu'aurait apporté l'utilisation successive d'un produit de rasage très lubrifiant, capable d'assurant une parfaite protection de l'épiderme pendant la durée de l'opération de rasage et le rasage terminé, elle apporte le bénéfice d'hydratation, de douceur propre à l'application sur l'épiderme d'un après-rasage.

4. Préparation, selon la revendication 2, **caractérisée par** sa formule, anhydre, (c'est-à-dire contenant moins de 2% de son poids en eau), hydrosoluble et non-moussante.

5. Préparation, selon la revendication 2, **caractérisée par le fait que** sa solution aqueuse mouillante et lubrifiante, mise en place sur le visage ou sur les parties à raser, imprègne aussi la bouillie de poils coupés qui se retrouve dans la tête de rasage du rasoir, dès lors le nettoyage des têtes de rasage s'effectue facilement par simple rinçage à l'eau.

6. Préparation, selon la revendication 2, **caractérisée par** l'effet substantif avec l'épiderme de ses composés lubrifiants, lequel fait qu'ils demeurent sur l'épiderme pendant toute la durée du rasage et que si la lubrification vient à diminuer, du fait de la perte d'eau liée aux conditions de rasage, il suffit alors de remouiller le visage en exerçant un petit massage de l'épiderme de quelques seconde pour recréer une bonne lubrification et de mener ainsi le rasage en gardant sur toute sa durée une bonne protection lubrifiante.
